# EUROPEAN PATENT APPLICATION

(11) **EP 4 736 926 A1**
(43) Date of publication of application: **06.05.2026**
(21) Application number: 24211017.9
(22) Date of filing: 05.11.2024
(51) Int. Cl.: A61M 25/00

(54) **DEVICE FOR DELIVERING OBJECTS INTO A SUBJECT**

(71) Applicant: Landers, Fabian Christopher, 8005 Zürich (CH); Pané, Salvador, 8057 Zürich (CH); Nelson, Bradley James, 8126 Zumikon (CH)
(72) Inventor: LANDERS, Fabian Christopher, 8005 Zürich (CH); SALVADOR, Pané, 8057 Zürich (CH); BRADLEY, James Nelson, 8126 Zumikon (CH); RISS, Silas, 9230 Flawil (CH)
(74) Representative: Kasche & Partner

(57) **Abstract**

Embodiments pertain to a device (2000) configured to transport objects (500) from outside a subject's body to at least one site of interest internal to a subject's body for delivering the objects at the at least one site of interest. The device comprises a device body (1000) having a device distal portion (1200) and a device proximal portion (1100). The device proximal portion includes a receptacle (1110) configured to receive the objects. The receptacle may be reversibly configurable from a closed configuration for securely holding the objects, to an open configuration for releasing the objects from the receptacle to the at least one site. The objects to be released can include, for example, hydrogel-particles, hydrogel-based particles, and/or nanoparticles, optionally encapsulated by hydrogel.

## Description

### BACKGROUND

Over the last century, the development of therapeutics such as chemotherapies, antibiotics, T-cell therapies, vaccinations, and cell and gene therapies has revolutionized medicine, significantly improving patient outcomes across a wide range of diseases. However, a critical trade-off must be made when administering therapeutic agents: the dose must be high enough to achieve a therapeutic effect but low enough to minimize side effects. This balance, known as the therapeutic window, defines the safe and effective dose range. Currently, most drugs are administered systemically, for example intravenously, resulting in a significant proportion of the drug failing to reach the target site. This inefficiency leads to off-target effects and severely restricts the therapeutic window. As a consequence, severe side effects accompany many treatments, and several promising drug candidates are rejected during clinical trials due to severe adverse effects. Limited targeting also inhibits many promising complex drugs, such as cell and gene therapies, which cannot be delivered to their target sites in adequate concentrations.

The description above is presented as a general overview of related art in this field and should not be construed as an admission that any of the information it contains constitutes prior art against the present patent application.

### BRIEF DESCRIPTION OF THE FIGURES

The figures illustrate generally, by way of example, but not by way of limitation, various embodiments discussed in the present document.

For simplicity and clarity of illustration, elements shown in the figures have not necessarily been drawn to scale. For example, the dimensions of some of the elements may be exaggerated relative to other elements for clarity of presentation. Furthermore, reference numerals may be repeated among the figures to indicate corresponding or analogous elements. References to previously presented elements are implied without necessarily further citing the drawing or description in which they appear. The figures are listed below.
**Figure 1** is a schematic illustration of a device comprising a receptacle in a retracted configuration, according to some embodiments.
**Figure 2** is a schematic illustration of the device in the expanded configuration, according to some embodiments.
**Figure 3** is a schematic illustration of a manufacturing process of the device, according to some embodiment.
**Figure 4** is a schematic illustration device prototypes (S1-S4) that may be obtained using different mold shapes, respectively, according to some embodiments.
**Figure 5A** shows a matrix of mold types S1-S4 and various cutting structures employed for obtaining Prototypes 1 using material PU 80A, according to some embodiments.
**Figure 5B** shows a matrix of mold types **S1-S4** and various cutting structures employed for obtaining Prototypes 1 using material PU 80B, according to some embodiments.
**Figures 6A** and **6B** show Prototypes made of PU30A, with three digits and **S3** and **S4** molds, according to some embodiments.
**Figure 7** shows high-density polyethylene (HDPE) medical tubes where a surgical scalpel was used to cut the individual digits, according to some embodiments.
**Figure 8** shows a prototype made of a Polyether ether ketone (PEEK) tube, according to some embodiments.
**Figure 9A-9C** showing a connector employed for connecting between a guidewire and the device, according to some embodiments.
**Figure 10** shows an image of release of a polymeric capsule in a Silicone Model, according to some embodiments.
**Figures 11A** and **11B** show a handle configured to controllably block and unblock passage of the device through a catheter, according to some embodiments.

### DESCRIPTION

Embodiments of the invention to a device configured to delivery drugs to a patient site of interest internal to a subject (e.g., a human and/or animal subject).

In some embodiments, the device may be configured to transport objects (also: payload) from outside a subject's body to a site of interest internal to a subject's body for delivering the objects to and releasing the objects at the site of interest. In some examples, the site of interest may be a treatment or target site. In some other examples, the objects may be transported from the site of interest to the target site by the flow of bodily fluid (e.g., blood stream) and/or other kinetic energy imparted on the objects released at the site of interest. The site of interest may include, for example, an intravenous, intraarterial, epidural, and/or intracranial (e.g., intrathecal, subarachonoate) space.

Objects deliverable by the device may include, for example, hydrogel-particles, hydrogel-based particles, and/or nanoparticles, optionally encapsulated by hydrogel, and/or microrobots for targeted therapies. Such microrobots may have a microrobotic (e.g., hydrogel-based) capsule configured for acting, for example, in mammalian vessels, cerebral spine, or subarachonoate space to act as an untethered end-effector. In some examples, such microrobotic end-effector may comprise a thermoresponsive hydrogel framework incorporating magnetic and radiopaque nanoparticles. Such microrobot may allow precise tracking and navigation thereof even at flow speeds up to, for example, 80 cm/s. Upon reaching a target site, the object (e.g., microrobot) can be remotely dissolved, e.g., through hyperthermic heating and/or ultrasound actuation, facilitating precise drug release. Accordingly, in some examples, the objects deliverable by the device may be dissolvable through remotely and/or on-site emitted energy.

The device, which may have a generally longitudinal axis, may have a device body comprising a device distal portion and a device proximal portion. The device proximal portion comprises a receptacle configured to receive and hold objects.

The term "proximal" as used herein, refers to a location or position that is, when operably engaged, closer to the subject or the site of interest. Correspondingly, the term "distal" as used herein refers to a location or position, during operable engagement of the device, further away from the subject or the site of interest.

The receptacle may be reversibly configurable from a closed configuration for securely holding the objects, to an open (also: expanded) configuration for releasing the objects from the receptacle to the at least one site of the interest. The receptacle is configured to securely transport the objects to and subsequent release the objects at the site of interest without damaging the structural integrity of most or all of the objects during their transport and release, to ensure achieving the desired therapeutic effect.

In some examples, in the closed configuration, the receptacle may be fluid-tight or sealed.

In some examples, the receptacle may be made of material having a hardness and/or rigidity that allows to securely hold and release the hydrogel particles without damaging the structural integrity of most or all of the hydrogel particles to be released.

In some examples, the receptacle may be made of material having a hardness and/or rigidity that allows to securely hold and release the hydrogel particles without adversely affecting the therapeutic effectiveness of the objects to be released to the at least one site of interest.

In some examples, the receptacle may comprise a cover (e.g., reversibly) configurable from a closed configuration for retaining the objects in the receptacle, to an open configuration for releasing at least some of the objects for delivery to the at least one site of interest.

In some examples, the receptacle may comprise a plurality of prongs (also: digits) extending proximally from a base portion and which are selectively configurable from the closed configuration for holding the objects to the open configuration for releasing at least some of the objects to the at least one site of interest.

In some examples, the receptacle may comprise a basket that is selectively configurable from the closed configuration for holding the objects to the open configuration for releasing at least some of the objects to the at least one site of interest.

In some examples, the receptacle may have a mesh-like structure. In some examples, the prongs and/or the basket may have a mesh-like structure.

In some examples, the receptacle may have a solid (e.g., sheet-like) structure. For example, the prongs and/or the basket may have a solid (e.g., sheet-like) structure.

In some examples, in the open configuration, the prongs are expanded away from each other, e.g., radially distanced away from a longitudinal axis of the device. In some embodiments, the prongs may be curved concave towards the longitudinal axis.

In some examples, a deployment system may be employed for transporting the device, along with the objects being held therein, from outside the subject to, nearby or adjacent to the site of interest internal to the subject.

The device deployment system may comprise a delivery catheter having a distal catheter opening and a proximal catheter opening. The catheter may be configured to slidably receive the device, and a guidewire for guiding the device from the distal catheter opening to the proximal catheter opening. The system may further include a guidewire for guiding, under vision, the proximal catheter opening from outside the subject to the site of interest internal to the subject. Once the proximal catheter has reached the site of interest, the guidewire is removed from within the catheter and the device is guided (e.g., pushed) via the catheter to the proximal catheter end to the site of interest for releasing of the at least one object to the site of interest.

Upon delivery of the objects, the receptacle may be retracted back via the proximal catheter opening into the catheter.

In some examples, the device may be configured such that the receptacle can be actuated from the closed to the open configuration, and back to the closed configuration.

In some examples, the device may be configured to retrieve objects (e.g., blood clots) from the site of interest from within the subject to outside the subject.

In some example, the device may be configured such that pushing the receptacle out from the proximal catheter opening causes the receptacle to assume the open configuration. In other words, the receptacle may be configured to automatically assume the open configuration when being pushed out of the proximal catheter opening. For instance, portions of the receptacle (e.g., a cover) may be prestressed such to assume, by default, the open configuration, in which the receptacle is relaxed. Retracting the receptacle back into the catheter may force the receptacle to assume again the closed configuration to fit into the catheter lumen.

In some examples, the receptacle may be actuated to assume the open and/or the closed configuration.

For instance, the receptacle may include material having superelastic characteristics such as, for example, shape-memory alloy (SMA). In some examples, the SMA may be thermally actuatable to assume the expanded configuration in an austenite state. In some examples, the receptacle may be thermally actuatable to assume again the closed configuration in an austenite state.

In some examples, the receptacle may be configured such to remain undamaged during retraction back into catheter for closing the receptacle. For example, the prongs may remain undamaged during their retraction into the catheter lumen (or any other external structure) imparting a radial force onto the prongs causing them to assume again the closed configuration.

In some examples, at least one prong of the plurality of prongs has a width that is at least 5 wider than a thickness of the prong.

In some examples, at least one prong of the plurality of prongs has a width that is at least 10 times wider than a thickness of the same prong.

In some examples, the device may comprise a handle for allowing controllably opening of the receptacle for releasing the at least one object. The handle may actuated to cause the receptacle to slide out of the catheter opening.

In some examples, the device may comprise polymeric material. In some examples, the device may comprise biocompatible material.

In some examples, the delivery catheter is an off-the-shelve delivery catheter.

In some examples, with respect to the subject's location, the device may be locally operable and/or deployable (e.g., by a clinician) and/or a remotely operable (e.g., robotically).

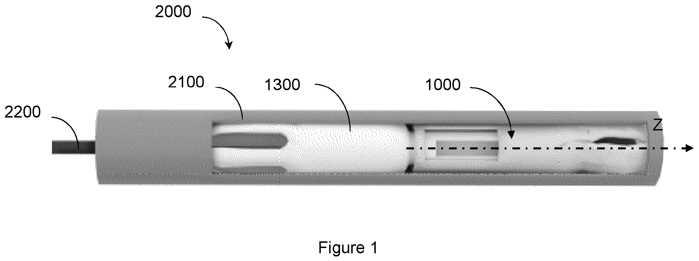

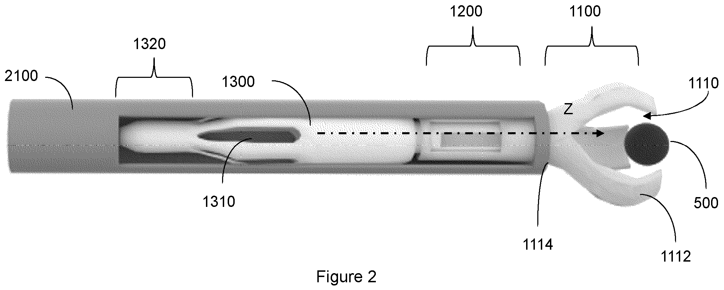

Reference is now made to **Figuresa 1 and 2.** In some embodiments, a delivery device **1000** may have a proximal delivery device portion **1100** and a distal delivery device portion **1200.** Proximal device portion **1100** may include a receptacle **1110** configured to carry a payload **500** and which may for example, in the form of a plurality of prongs **1112,** extending proximally from a base portion **1114.** Receptacle **1110** may be configurable from a closed configuration (**Figure 1**) to an open configuration **(****Figure 2****).**

In some embodiments, a deployment system **2000** may be employed for transporting delivery device **1000** to the site of interest. Such deployment system **2000** may for example include a catheter **2100** and a guidewire **2200.**

In some embodiments, once delivery device **1000** is pushed out of catheter **2100,** the prongs or digits **1112** may assume their relaxed configuration allowing release of payload 500.

In some examples, to attain desired characteristics, prongs, which may be made of polymeric material, may be reinforced with a metallic wire, e.g., to obtain desired elastic characteristics.

In some embodiments, delivery device **1000** may further include a connector **1300** configured to be operably coupleable with guidewire **2200** of deployment system **2000** such that pushing guidewire **2200** connected with connector **1300** allows pushing device **1000** towards the proximal catheter opening.

In some embodiments, base **1114** may have an opening such that the receptacle cavity may be configured to be in fluid communication with the catheter lumen for allowing pressured fluid to flow from the catheter distal end towards to the proximal end to enter the receptacle cavity. The flow of fluid may assist in imparting energy onto the payload **500** to facilitate delivery of payload **500** at the site of interest.

In some embodiments, connector **1300** may have at least one opening **1310** for bringing the distal catheter end and the internal lumen of connector **1300** in fluid communication with each other. In some embodiments, connector **1300** may be shaped such that fluid surrounding guidewire **2200** flows from around a distal portion **1320** of connector **1300** and enters the at least one connector opening **1310.** In some embodiments, the at least one connector opening **1310** may be longitudinal, extending in the axial direction **Z.** In some embodiments, a plurality of connector openings **1310** may be radially arranged for allowing fluid to enter the connector lumen. In some examples, the outer walls of the **portion** comprising connector openings **1310** may configured to abut against the inner wall of catheter **2100,** e.g., to create seal between the delivery device distal portion **1200** and the distal catheter end. This way, most or all of the fluid flowing in the catheter in proximal direction is forced to enter the connector lumen. The connector may further comprise a proximal opening which is in fluid communication with the receptacle's base opening.

This way, fluid may flow from catheter **2100** through connector **1300** into receptacle cavity to facilitate delivery of payload **500** at the site of interest.

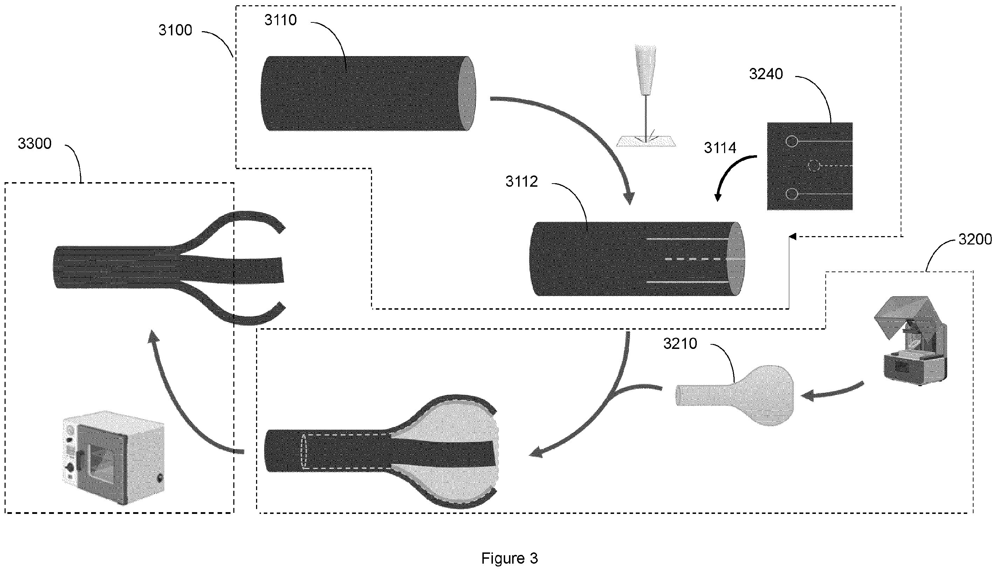

Additional reference is made to **Figure 3****.** In some embodiments, device **1000** may be manufactured by employing structure cutting (step **3100**) for cutting an initial structure **3110** into a desired structure **3112,** mold preparation (step **3200**) for obtaining a mold **3210,** and by employing thermo-molding (step **3300**)**.** In some examples, structure cutting (**3100**) may include adding an auxiliary structure **3240** (step **3114**) for preventing crack propagation of the ends of the individual digits **1112.** Optionally, a knife or any other suitable can be used for cutting the structures, instead of the laser mill.

### Example fabrication processes:

**Structure cutting:** A QuickLaze-50 ST2 laser mill (New Wave Research Co. Ltd.; 8 Avo Cour Ermine Business ParkHuntingdon, Cambswas; England UK PE29 6XS) was used to cut the individual digits of various tube-shaped materials (see prototypes) and shape the ends of the digits. Optionally, a structure for preventing crack propagation of the ends of the individual digits can be added. Optionally, a knife can be used for cutting the structures, instead of the laser mill.

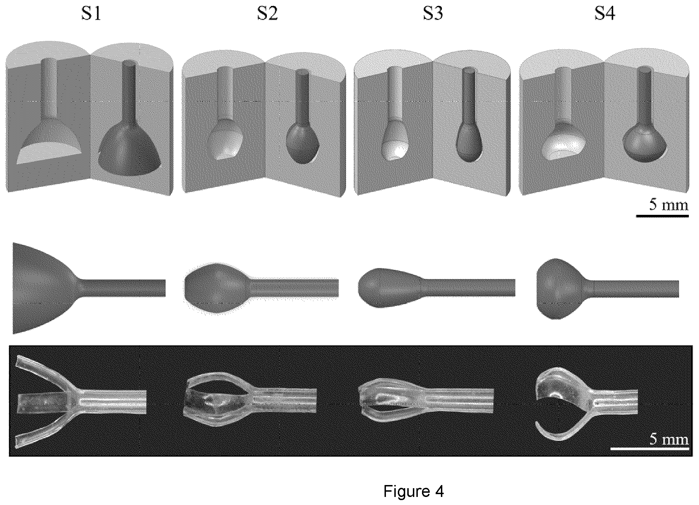

**Mold preparation:** 3D molds (negative and positive as in the figure below) were created in Solidworks (Dassault Systèmes SolidWorks Corporation; 175 Wyman Street; Waltham, MA 0245) and 3D printed using a Phrozen Sonic Mini 8K (PHROZEN TECH CO., LTD.; 3F., NO.287, NIUPU RD., XIANGSHAN DIST., HSINCHU CITY 30091, TAIWAN (R.O.C.)) and Porhzen Aqua 4k Ivory resin (PHROZEN TECH CO., LTD.; 3F., NO.287, NIUPU RD., XIANGSHAN DIST., HSINCHU CITY 30091, TAIWAN (R.O.C.)). The digits of the tube were then fixed on the mold between the negative and the positive structure. Several mold forms were used, resulting in several gripper shapes (**Figure 4** schematically shows several device prototypes obtained using different mold shapes **S1-S4)**

**Thermo molding:** The entire structure was put into an oven and heated above a specific temperature, causing the polymer to retain the shape of the mold.

In the discussion, unless otherwise stated, adjectives such as "substantially" and "about" that modify a condition or relationship characteristic of a feature or features of an embodiment of the invention, are to be understood to mean that the condition or characteristic is defined to within tolerances that are acceptable for operation of the embodiment for an application for which it is intended.

### Example Prototypes 1

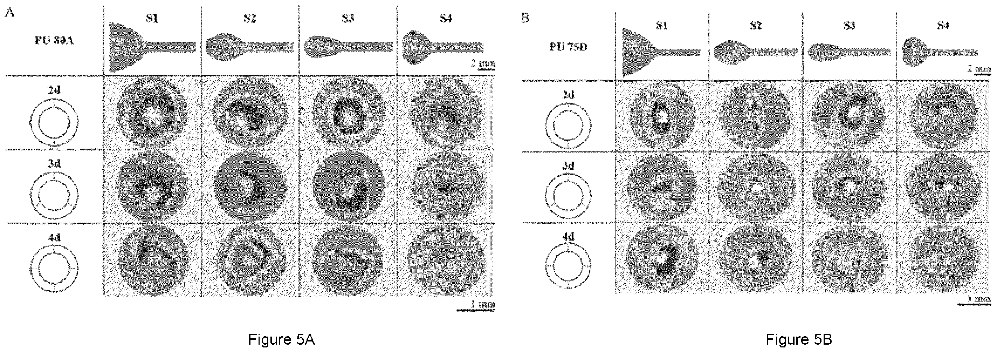

Reference is made to **Figures 5A** and **5B****.** Prototypes 1 were fabricated from a Polyurethan 80 A (inner diameter 0.047 inch, outer diameter 0.058 inch) and Polyurethan 75 A (inner diameter 0.031 inch, outer diameter 0.056 inch) medical tube (GenX Medical, L.L.C.; 4701 Wilson Rd, Bldg A, Chattanooga, TN 37410). The tube was cut with a laser wavelength of 355 nm. Several numbers of digits and mold geometries were fabricated and thermoformed at 120°C for 30 min. **Figure 5A** shows a matrix of mold types S1-S4 and various cutting structures employed for obtaining Prototypes 1 using material PU 80A. **Figure 5B** shows a matrix of mold types **S1-S4** and various cutting structures employed for obtaining Prototypes 1 using material PU 80B.

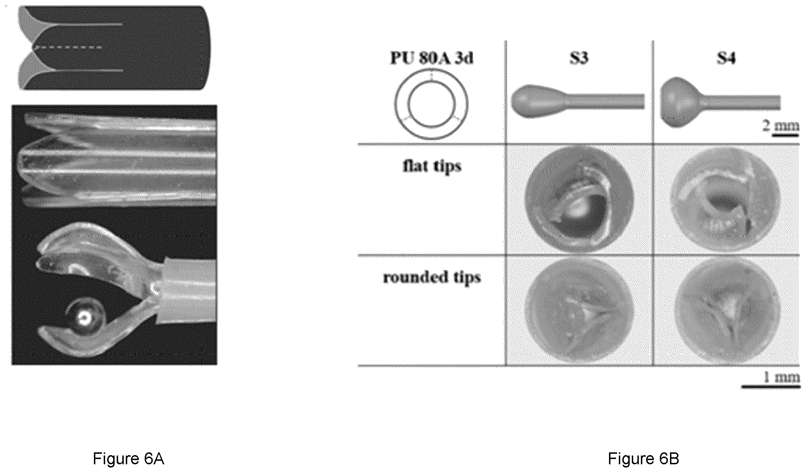

Further reference is made to **Figures 6A** and **6B****.** The superior performance of PU30A, with three digits and S3 and S4 molds, was determined. In the next iteration, the tips of these digits were rounded for superior sealing or shielding behaviour. The figure below shows that the mold shape S4 with rounded digits yielded good sealing and protective performance.

### Example Prototype 2

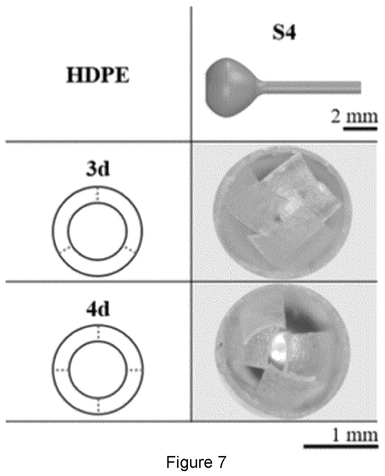

Additional reference is made to **Figure 7****.** In another prototype, high-density polyethylene (HDPE) medical tubes (inner diameter 0.0287 inches, outer diameter 0.0335 inches) (GenX Medical, L.L.C.; 4701 Wilson Rd, Bldg A, Chattanooga, TN 37410) were used. A surgical scalpel (Balde No. 3, Handle No. 3; Swann-Morton Limited, Owlerton Green; Sheffield; S6 2BJ) was used instead of the lasermill to cut the individual digits. An S4-shaped mold was used with three and four digits at 120°C for 25 min, with the three-digit prototype resulting in good sealing and protective behaviour.

### Example Prototype 3

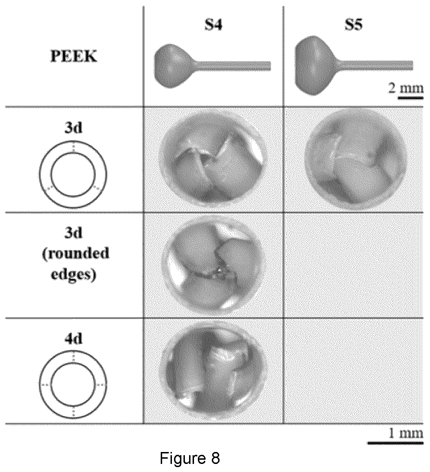

Further reference is made to **Figure 8****.** In the third prototype, a Polyether ether ketone (PEEK) tube (inner diameter 0.03 inch, outer diameter 0.04 inch) (Nordston medical; Ballybrit Upper Industrial Estate; Monivea Road; H91 XP49, Galway; Ireland) was selected for its superior stiffness and thermo molding properties. The laser mill was utilized at 532 nm to cut the tube. Next, the digits were molded into shape at 160°C for 120 min, resulting in grippers with good sealing capacity.

### Guidewire connector

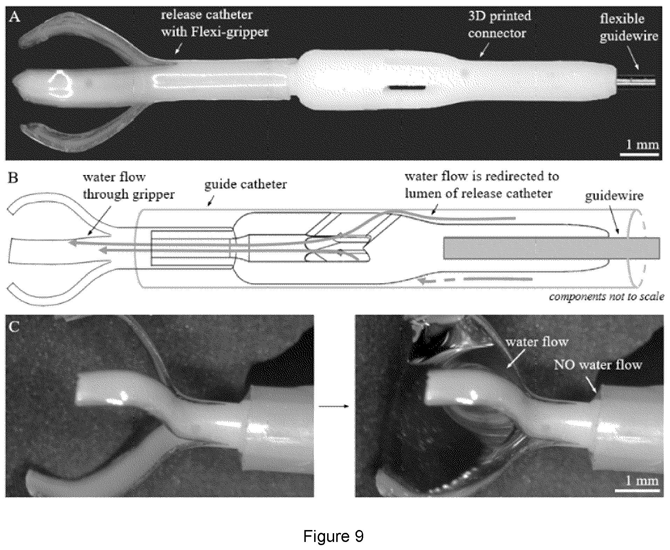

Reference is now made to **Figure 9A-9C**. In a clinical application, a guidewire may be used to advance the gripper through the guide catheter. For such setups, we developed a custom connector that connects the gripper to the guidewire. It was designed using Solidworks (Dassault Systèmes SolidWorks Corporation; 175 Wyman Street; Waltham, MA 0245) and 3D printed using a Phrozen Sonic Mini 8K (PHROZEN TECH CO., LTD.; 3F., NO.287, NIUPU RD., XIANGSHAN DIST., HSINCHU CITY 30091, TAIWAN (R.O.C.) and Porhzen Aqua 4k Ivory resin (PHROZEN TECH CO., LTD.; 3F., NO.287, NIUPU RD., XIANGSHAN DIST., HSINCHU CITY 30091, TAIWAN (R.O.C.). Notably, the connector can be hollow and have fluid inlets that permit fluid-flushing through the guide catheter and out of the center of the gripper. This may be used to aid the payload release process. Further, the connector can be fabricated via many different methods, such as injection molding or C.N.C. machining, and can be coated with biocompatible materials.

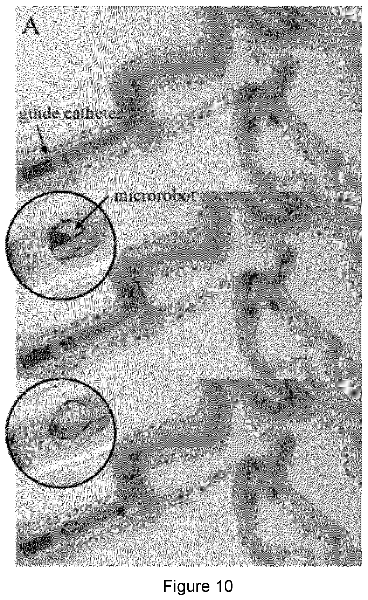

Reference is now made to **Figure 10****,** showing an image of release of a polymeric capsule in a Silicone Model (Swiss Vascular; Gotthardstrasse 25; 8002 Zurich) using the connector, prototype 3, and commercial guide wire (X-pedition hydrophobic guide wire, Medtronic; Medtronic (Schweiz) A.G.;Weltpoststrasse 5;3015 Bern; Switzerland).

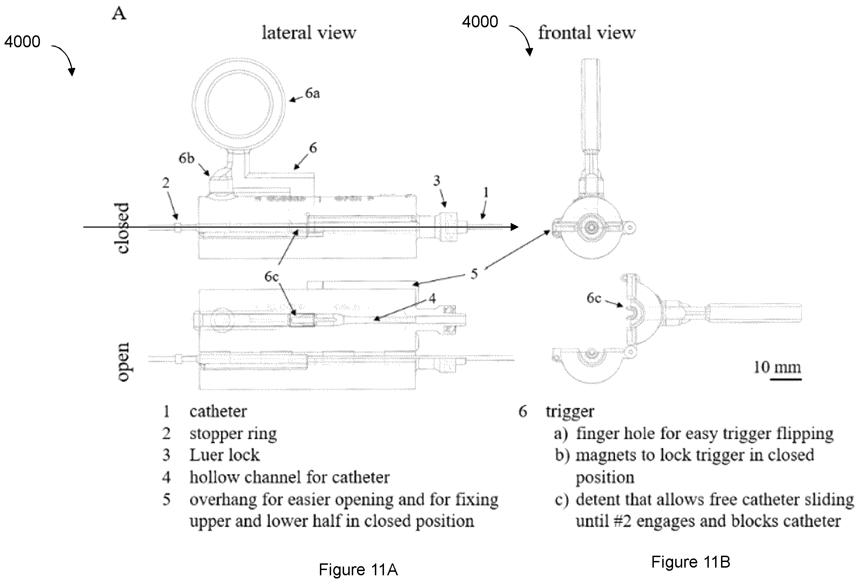

Additional reference is made to **Figures 11A** and **11B****.** In some embodiments, a release system **4000** may be employed that is configured to allow selectively (also: controllably) block and unblock passage of guidewire **2100** through catheter **2100.** Release system **4000** may for example be employed as a safety mechanism, preventing from an operator to unintentionally cause device **1000** to be pushed out of the proximal catheter end at an undesired location.

In some embodiments, release system **4000** may include a first stopper element **6c** (e.g., detent) that may be configured to cooperatively engage with and disengage from a second stopper **2** that may optionally be arranged on guidewire **2100.** When handle **6a** of release system **4000** is in a first (blocking) position, the first stopper may be in a position in which the second stopper may operably engage with the second stopper such to prevent passage of device **1000** through the tube of the release system. When handle **6a** of release system **4000** is put in a second (unblocking) position, the second stopper may pass through the system's lumen such that the guidewire can be further pushed in proximal direction towards the site of interest.

To trigger the gripper's opening (i.e., cargo release), a handheld release system was developed that stops the gripper before it is pushed over the delivery catheter. After opening a handle (6), the gripper can be pushed out of the delivery catheter, releasing the cargo.

The entire release handle was designed using Solidworks (Dassault Systèmes SolidWorks Corporation; 175 Wyman Street; Waltham, MA 0245) and 3D printed using a Phrozen Sonic Mini 8K (PHROZEN TECH CO., LTD.; 3F., NO.287, NIUPU RD., XIANGSHAN DIST., HSINCHU CITY 30091, TAIWAN (R.O.C.) and Porhzen Aqua 4k Ivory resin (PHROZEN TECH CO., LTD.; 3F., NO.287, NIUPU RD., XIANGSHAN DIST., HSINCHU CITY 30091, TAIWAN (R.O.C.)

Unless otherwise specified, the terms 'about' and/or 'close' with respect to a magnitude or a numerical value may imply to be within an inclusive range of -10% to +10% of the respective magnitude or value.

### Additional examples:

Embodiments pertain to a device configured to transport objects from outside a subject's body to at least one site of interest internal to a subject's body for delivering the objects at the at least one site of interest, the device comprising:
a device body comprising a device distal portion and a device proximal portion;
the device proximal portion comprising a receptacle configured to receive the objects;
wherein the receptacle is reversibly configurable from a closed configuration for securely holding the objects, to an open configuration for releasing the objects from the receptacle to the at least one site;
wherein the objects include hydrogel-particles, hydrogel-based particles, and/or nanoparticles, optionally encapsulated by hydrogel; and
wherein the receptacle is configured to securely hold the particles for transport to and delivery at the at least one site of interest without damaging the structural integrity of most or all of the particles.

In some examples, the receptacle is made of material having a hardness and/or rigidity or stiffness that allows to securely hold and release the hydrogel particles without damaging the structural integrity of most or all of the hydrogel particles to be released. Material stiffness for instance may range, for example, from 0-75 GPa, from 0-70 GPa, or from 0-30 GPa, or from 0-10 GPa.

In some examples, the receptacle is made of material having a hardness and/or rigidity that allows to securely hold and release the hydrogel particles without adversely affecting the therapeutic effectiveness of the objects released to the at least one site of interest.

In some examples, the receptacle comprises a cover configurable from a closed configuration for retaining the objects in the receptacle, to an open configuration for releasing at least some of the objects for delivery to the at least one site of interest.

In some examples, the receptacle comprises a plurality of prongs which are selectively expandable from the closed configuration for holding the objects to the open configuration for releasing at least some of the objects to the at least one site of interest wherein, optionally, in the open configuration, the prongs are expanded or distanced away from each other.

In some examples, the receptacle is configurable from the open to the closed configuration.

In some examples, the prongs and/or the catheter are configured such to remain undamaged during retraction into catheter, and/or wherein the prongs and/or the catheter are configured such to remain undamaged during retraction back into catheter.

In some examples, at least one prong of the plurality of prongs has a width that is at least 5 wider than a thickness of the prong, or wherein at least one prong of the plurality of prongs has a width that is at least 10 times wider than a thickness of the same prong.

In some examples, the device has a generally longitudinal axis.

In some examples, the device body is slidably receivable by a delivery catheter having a distal catheter end and a proximal catheter end,

the proximal catheter end having a proximal catheter opening through which the receptacle can be pushed, to allow the receptacle to assume the open configuration for releasing of the at least one object to the site.

In some examples, the receptacle is configured to automatically assume the open configuration when being pushed out of the proximal catheter opening.

In some examples, the prongs and/or cover are configured to automatically assume the expanded or open configuration when being pushed out of the proximal catheter opening.

In some examples, the device comprises a handle for allowing controllably opening of the receptacle for releasing the at least one object, wherein, optionally, the handle can be actuated to cause the receptacle to slide out of the catheter opening.

In some examples, the receptacle comprises a shape-memory alloy (SMA) or other superelastic material. In some embodiments, SMA may be employed for thermal actuation for the receptacle to assume the expanded configuration in an austenite state.

In some examples, the device comprises polymeric material.

In some examples, the device comprises biocompatible material.

In some examples, the delivery catheter is an off-the-shelve delivery catheter.

In some examples, the handle is operably engageable by a robot and/or by a human operator.

In some examples, the at least one site of interest includes any one or more of the following: mammalian vessels, cerebral spine, or subarachnoid space.

Unless otherwise specified, the terms 'about' or 'close' imply at or in a region of, or close to a location or a part of an object relative to other parts or regions of the object.

Positional terms such as "upper", "lower" "right", "left", "bottom", "below", "lowered", "low", "top", "above", "elevated", "high", "vertical" and "horizontal" as well as grammatical variations thereof as may be used herein do not necessarily indicate that, for example, a "bottom" component is below a "top" component, or that a component that is "below" is indeed "below" another component or that a component that is "above" is indeed "above" another component as such directions, components or both may be flipped, rotated, moved in space, placed in a diagonal orientation or position, placed horizontally or vertically, or similarly modified. Accordingly, it will be appreciated that the terms "bottom", "below", "top" and "above" may be used herein for exemplary purposes only, to illustrate the relative positioning or placement of certain components, to indicate a first and a second component or to do both.

"Coupled with" means indirectly or directly "coupled with".

It is important to note that the method may include is not limited to those diagrams or to the corresponding descriptions. For example, the method may include additional or even fewer processes or operations in comparison to what is described herein. In addition, embodiments of the method are not necessarily limited to the chronological order as illustrated and described herein.

It should be understood that where the claims or specification refer to "a" or "an" element and/or feature, such reference is not to be construed as there being only one of that element. Hence, reference to "an element" or "at least one element" for instance may also encompass "one or more elements".

As used herein the term "configuring" and/or 'adapting' for an objective, or a variation thereof, implies using materials and/or components in a manner designed for and/or implemented and/or operable or operative to achieve the objective.

Unless otherwise stated or applicable, the use of the expression "and/or" between the last two members of a list of options for selection indicates that a selection of one or more of the listed options is appropriate and may be made, and may be used interchangeably with the expressions "at least one of the following", "any one of the following" or "one or more of the following", followed by a listing of the various options.

As used herein, the phrase "A,B,C, or any combination of the aforesaid" should be interpreted as meaning all of the following: (i) A or B or C or any combination of A, B, and C, (ii) at least one of A, B, and C; and (iii) A, and/or B and/or C. This concept is illustrated for three elements (i.e., A,B,C), but extends to fewer and greater numbers of elements (e.g., A, B, C, D, etc.).

It should be appreciated that certain features, structures, characteristics, stages, methods, modules, elements, entities or systems disclosed herein, which are, for clarity, described in the context of separate examples, embodiments and/or the like, may also be provided in a suitable combination in a single example, embodiment, and/or the like. Conversely, various features, structures, characteristics, stages, methods, modules, elements, entities or systems disclosed herein, which are, for brevity, described in the context of a single example and/or embodiment, may also be provided separately or in any suitable sub-combination.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

While the invention has been described with respect to a limited number of embodiments, it will be appreciated that many variations, modifications, and other applications of the invention can be made within the scope of the appended claims.

## Claims

1. A device configured to transport objects from outside a subject's body to at least one site of interest internal to a subject's body for delivering the objects at the at least one site of interest, the device comprising:
a device body comprising a device distal portion and a device proximal portion;
the device proximal portion comprising a receptacle configured to receive the objects;
wherein the receptacle is reversibly configurable from a closed configuration for securely holding the objects, to an open configuration for releasing the objects from the receptacle to the at least one site;
wherein the objects include hydrogel-particles, hydrogel-based particles, and/or nanoparticles, optionally encapsulated by hydrogel; and
wherein the receptacle is configured to securely hold the particles for transport to and delivery at the at least one site of interest without damaging the structural integrity of most or all of the particles.

2. The device of claim 1, wherein the receptacle is made of material having a hardness and/or rigidity that allows to securely hold and release the hydrogel particles without damaging the structural integrity of most or all of the hydrogel particles to be released.

3. The device of claim 1 and/or claim 2, wherein the receptacle is made of material having a hardness and/or rigidity that allows to securely hold and release the hydrogel particles without adversely affecting the therapeutic effectiveness of the objects released to the at least one site of interest.

4. The device of any one or more of the preceding claims, wherein the receptacle comprises a cover configurable from a closed configuration for retaining the objects in the receptacle, to an open configuration for releasing at least some of the objects for delivery to the at least one site of interest.

5. The device of any one or more of the preceding claims, wherein the receptacle comprises a plurality of prongs which are selectively expandable from the closed configuration for holding the objects to the open configuration for releasing at least some of the objects to the at least one site of interest wherein, optionally, in the open configuration, the prongs are expanded or distanced away from each other.

6. The device of any one or more of the preceding claims, wherein the receptacle is configurable from the open to the closed configuration.

7. The device of any one or more of the claims 5 and/or 6, wherein the prongs and/or the catheter are configured such to remain undamaged during retraction into catheter, and/or wherein the prongs and/or the catheter are configured such to remain undamaged during retraction back into catheter.

8. The device of any one or more of the claims 4 to 7, wherein at least one prong of the plurality of prongs has a width that is at least 5 wider than a thickness of the prong, or wherein at least one prong of the plurality of prongs has a width that is at least 10 times wider than a thickness of the same prong.

9. The device of any one or more of the preceding claims, having a generally longitudinal axis.

10. The device of any one or more of the preceding claims, wherein the device body is slidably receivable by a delivery catheter having a distal catheter end and a proximal catheter end,
the proximal catheter end having a proximal catheter opening through which the receptacle can be pushed, to allow the receptacle to assume the open configuration for releasing of the at least one object to the site.

11. The device of any one or more of the preceding claims, wherein the receptacle is configured to automatically assume the open configuration when being pushed out of the proximal catheter opening.

12. The device of any one or more of the claims 5 to 10, wherein the prongs and/or cover are configured to automatically assume the expanded or open configuration when being pushed out of the proximal catheter opening.

13. The device of any one or more of the preceding claims, comprising a handle for allowing controllably opening of the receptacle for releasing the at least one object, wherein, optionally, the handle can be actuated to cause the receptacle to slide out of the catheter opening.

14. The device of any one or more of the preceding claims, wherein the receptacle has superelastic characteristics.

15. The device of any one or more of the claims 9 to 14, wherein the delivery catheter is an off-the-shelve delivery catheter.
